Europäisches Patentamt

European Patent Office

Office européen des brevets

(19)

(11) **EP 1 423 675 B1**

(12) **EUROPÄISCHE PATENTSCHRIFT**

(45) Veröffentlichungstag und Bekanntmachung des Hinweises auf die Patenterteilung:
**29.03.2006 Patentblatt 2006/13**

(21) Anmeldenummer: **02760127.7**

(22) Anmeldetag: **21.08.2002**

(51) Int Cl.:
*G01N 11/00* (2006.01)   *G01N 33/28* (2006.01)

(86) Internationale Anmeldenummer:
**PCT/DE2002/003052**

(87) Internationale Veröffentlichungsnummer:
**WO 2003/019151 (06.03.2003 Gazette 2003/10)**

(54) **VERFAHREN UND VORRICHTUNG ZUR MESSUNG DER VISKOSITÄT EINER FLÜSSIGKEIT**

METHOD AND DEVICE FOR MEASURING THE VISCOSITY OF A LIQUID

PROCÉDÉ ET DISPOSITIF POUR MESURER LA VISCOSITÉ D'UN LIQUIDE

(84) Benannte Vertragsstaaten:
**DE FR SE SK TR**

(30) Priorität: **25.08.2001 DE 10141694**

(43) Veröffentlichungstag der Anmeldung:
**02.06.2004 Patentblatt 2004/23**

(73) Patentinhaber: **ROBERT BOSCH GMBH**
**70442 Stuttgart (DE)**

(72) Erfinder: **JAKOBY, Bernhard**
**72764 Reutlingen (DE)**

(56) Entgegenhaltungen:
**EP-A- 0 616 204**      **DE-A- 10 008 547**
**US-A- 3 479 863**      **US-A- 5 423 302**

- **PATENT ABSTRACTS OF JAPAN vol. 018, no. 420 (P-1782), 5. August 1994 (1994-08-05) & JP 06 129974 A (MATSUSHITA ELECTRIC IND CO LTD), 13. Mai 1994 (1994-05-13)**

**Beschreibung**

Stand der Technik

Es sind software-basierte Systeme zur Anzeige eines notwendigen Motorölwechsels bei Kraftfahrzeugen bekannt, von denen einige auf Algorithmen basieren, die Parameter wie etwa die seit dem letzten Ölwechsel zurückgelegte Kilometerfahrleistung oder die Häufigkeit von Kaltstarts auswerten.

Alternativ dazu wird bei anderen bekannten verfahren auf Sensorsignale zurückgegriffen, die direkt den physikalischen Ölzustand beschreiben, wobei beispielsweise durch geeignete Sensoren die Dielektrizitätszahl des Öls oder als weit zuverlässigere Größe die Ölviskosität gemessen werden kann. Aus der Bestimmung des Viskositätsveränderung des Motoröls seit dem letzten Ölwechsel kann dabei ein viskositätsbasiertes Ölwechselkriterium abgeleitet werden, da ein Motorölverschleiß üblicherweise mit einem Anstieg des Viskositätswertes verbunden ist. In einer Auswerteelektronik mit angeschlossener Anzeigevorrichtung kann bei herkömmlichen Systemen beispielsweise ein Viskositätsgrenzwert gespeichert werden, der mit den gemessen Viskositätswerten des Motoröls verglichen wird und bei dessen Überschreitung dem Fahrzeugführer ein Hinweis auf die Fälligkeit des nächsten Motorölwechsels gegeben wird.

Die bei diesen, aus dem Stand der Technik bekannten Verfahren ermittelten physikalischen Ölparameter sind in aller Regel temperaturabhängig, so dass zur Bestimmung eines Vergleichswertes eine Temperaturkompensationsberechnung notwendig ist. Weiterhin ist aus der unveröffentlichten Patentanmeldung DE 100 085 47 ein Verfahren zur Beurteilung des Verschleißes von Motoröl bekannt, bei dem durch einen Sensor die Ölviskosität des Motoröls gemessen wird und bei dem durch einen Sensor die Ölviskosität des Motoröls gemessen wird und bei dem dem Sensor zur Ölviskositätsmessung ein Temperaturmessfühler zur gleichzeitigen Bestimmung der Öltemperatur zugeordnet ist, wobei die Ölviskosität und die Öltemperatur in der Abkühlphase des Motors nach dessen Abstellen gemessen wird.

Aus der US 5,423,302 A ist ein Verfahren bekannt, bei dem zur Steuerung eines Injektors die Viskosität einer Flüssigkeit mittels eines Viskositätssensors erfasst wird. Um die Viskositätsschwankungen zu erfassen, die sich aufgrund von Temperaturänderungen ergeben, wird zusätzlich ein Temperatursensor verwendet.

Aus der US 3,479,863 A ist eine Vorrichtung bekannt, bei der die Temperaturabhängigkeit der Viskosität durch die Verwendung eines Temperatursensors und eines Viskositätssensors erfasst wird. Dabei ist vorgesehen, dass der beide Sensoren räumlich voneinander getrennt in die zu messende Flüssigkeit getaucht werden.

Vorteil der Erfindung

Das erfindungsgemäße Verfahren und die erfindungsgemäße Vorrichtung mit den Merkmalen der nebengeordneten Ansprüche hat demgegenüber den Vorteil, dass zu beliebigen Zeitpunkten, das heißt nicht lediglich in der Abkühlphase des Motors, die Ölviskosität gemessen werden kann. Weiterhin ist das erfindungsgemäße Verfahren und die erfindungsgemäße Vorrichtung dadurch ausgezeichnet, dass es in vorteihafterweise möglich ist, eine genauere Bestimmung der Viskosität durchzuführen. Bei der Überwachung von flüssigen Betriebsstoffen, wie zum Beispiel Motoröl, kann nämlich eine Viskositätsmessung mittels eines Viskositätssensors zur Beurteilung des aktuellen Flüssigkeitszustandes herangezogen werden. Da jedoch die Viskosität $\eta$ in der Regel eine stark temperaturabhängige Größe ist, ist es unerläßlich, gleichzeitig die Temperatur T durch einen Temperatursensor zu bestimmen. Ein entsprechend ermitteltes Viskositäts-Temperatur-Messwertepaar $\{\eta, T\}$ charakterisiert einen Punkt der Viskositäts-Temperatur-Charakteristik $\eta=\eta$ (T) der Flüssigkeit. Bei der Erfassung mehrerer Punkte bei verschiedenen Temperaturen - dies ist leicht möglich, wenn das zu überwachende Medium sich im Regelbetrieb erwärmt und abkühlt, wie das zum Beispiel beim Motoröl der Fall ist - können mehrere Messwertepaare zur Interpolation der Charakteristik $\eta$(T) verwendet werden. Das erfindungsgemäße Verfahren gemäß dem Hauptanspruch hat den Vorteil, dass die Viskositäts-Temperatur-Messwertepaare $\{\eta,T\}$ mit einer größeren Genauigkeit bestimmbar sind und somit zur genaueren Charakterisierung der Viskositäts-Temperatur-Charakteristik $\eta=\eta$ (T) herangezogen werden, da die von dem Viskositätssensor gemessene viskosität $\eta$ im allgemeinen bei einer von der vom Temperatursensor gemessenen Temperatur verschiedenen Temperatur ermittelt wurde. Die durch die räumliche Trennung von Viskositätssensor und Temperatursensor hervorgerufene unterschiedliche Temperatur der Flüssigkeit am Ort des Viskositätssensors und am Ort des Temperatursensors rührt von einem Temperaturgradienten in der Flüssigkeit her. Ein solcher Temperaturgradient tritt bevorzugt bei starker Wärmezufuhr bzw. Wärmeabfuhr in der zu überwachenden Flüssigkeit auf. Daher ist das erfindungsgemäße Verfahren und die erfindungsgemäße Vorrichtung insbesondere bei großen Temperaturgradienten vorteilhaft einsetzbar, weil dort die ohne das erfindungsgemäße Verfahren bzw. die erfindungsgemäße Vorrichtung durchgeführten Viskositätsmessungen besonders ungenau wären.

Durch die in den Unteransprüchen aufgeführten Maßnahmen sind vorteilhafte Weiterbildungen und Verbesserungen des im Hauptanspruch angegebenen Verfahrens und der im Nebenanspruch angegebenen Vorrichtung möglich.

Zeichnung

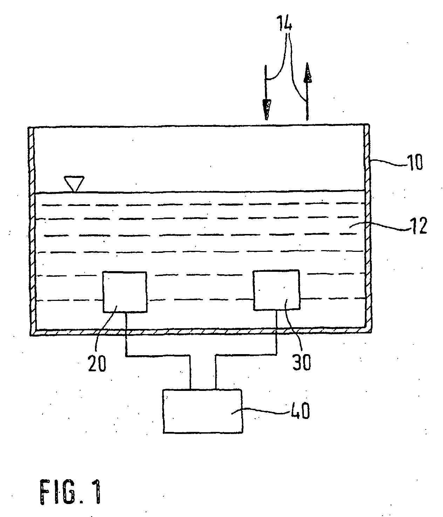

Ein Ausführungsbeispiel der Erfindung ist in der Zeichnung dargestellt und in der nachfolgenden Beschreibung näher erläutert. Es zeigt Figur 1 ein Prinzipschema der Messanordnung.

Beschreibung des Ausführungsbeispiels

In Figur 1 ist ein Behälter 10 mit einer Flüssigkeit 12 dargestellt. In dem Behälter 10 bzw. an dem Behälter 10 ist ein Viskositätssensor 20 und ein Temperatursensor 30 vorgesehen. Sowohl der Viskositätssensor 20 als auch der Temperatursensor 30 ist mit einer Auswerteeinheit 40 verbunden. Mittels zweier durch das Bezugszeichen 14 bezeichnete Pfeile wird angedeutet, dass es betriebsbedingt bei dem in Figur 1 dargestellten Behälter 10 zu einer Zufuhr und/oder zu einer Abfuhr von Wärme und/oder der Flüssigkeit 12 kommt. Weiterhin kann die Flüssigkeit 12 im Behälter 10 bewegt werden, beispielsweise durch Umwälzpumpen oder auch durch Konvektion, wobei aufgrund von besonders kalten oder besonders heißen Stellen des Behälters 10 ein Temperaturgradient in der Flüssigkeit 12 entsteht.

Wenn vorausgesetzt wird, dass in der Flüssigkeit 12 ein Temperaturgradient zu einem vorgegebenen Zeitpunkt t vorhanden ist, dann bewirkt die räumliche Trennung des Viskositätssensors 20 von dem Temperatursensor 30, dass die Temperatur der Flüssigkeit 12 an der räumlichen Position des Viskositätssensors 20 von jener an der Position des Temperatursensors 30 abweicht. Dementsprechend repräsentiert die von dem Temperatursensor 30 gemessene Temperatur $T_{30}$ nicht die (im Prinzip unbekannte) Temperatur $T_{20}$ der Flüssigkeit 12 an der Stelle des Viskositätssensors 20. Demnach können die Viskositäts-Temperatur-Messwertepaare $\{\eta, T_{30}\}$ a priori nicht fehlerfrei zur Charakterisierung der Viskositäts-Temperatur-Charakteristik $\eta=\eta$ (T) herangezogen werden, da die von dem Viskositätssensor 20 gemessene Viskosität $\eta$ im allgemeinen bei einer von $T_{30}$ verschiedenen Temperatur (nämlich $T_{20}$) ermittelt wurde. Die vorliegende Erfindung beschreibt jedoch ein Verfahren zur Ermittlung gültiger Messwertepaare.

Die angesprochenen Temperaturgradienten treten bevorzugt bei starker Wärmezufuhr bzw. Wärmeabfuhr in der zu überwachenden Flüssigkeit 12 auf. Diese Wärmeabfuhr bzw. Wärmezufuhr ist in Figur 1 mit dem Bezugszeichen 14 und den entsprechenden Pfeilen dargestellt. Bei Wegfall dieser Wärmezufuhr stabilisiert sich die Temperaturverteilung weitgehend durch thermische Ausgleichsvorgänge, so dass im Rahmen der angestrebten Genauigkeit die Gleichung gilt

$$T_{30}=T_{20}.$$

Erfindungsgemäß ist es vorgesehen, einen Algorithmus zu verwenden, welcher aus einer Reihe aufgezeichneter Messwerte bzw. Messwertepaare jene Werte herausfiltert, welche mit hinreichender Genauigkeit zur Ermittlung der Viskositäts-Temperatur-Charakteristik $\eta$ (T) verwendet werden können. Wenn von einer Aufzeichnung der Viskositätsmesswerte $\eta_k$ und der Temperaturmesswerte $T_{30,k}$ zu diskreten Zeitpunkten $t_k$ ausgegangen wird - wie dies beispielsweise durch einen Mikrocontroller in der Auswerteeinheit 40 durchführbar ist - dann können die zu einem vorgegebenen Zeitpunkt, beispielsweise zum Zeitpunkt $t_k$, zusammengehörigen Werte zu einem Messwertetripel $M_k=\{\eta_k, T_{30,k}, t_k\}$ zusammengefaßt werden, wobei der Index k sowohl bei $\eta$ als auch bei $T_{30}$ anzeigt, dass diese Messwerte zum k-ten Messzeitpunkt gemessen wurden. Im allgemeinsten Fall ermittelt der Algorithmus für das Messwertetripel, welches zum Zeitpunkt $t_k$ gehört, eine Maßzahl $Z_k$ bzw. ein Maß $Z_k$, welche(s) den Grad der Inhomogenität in der Temperaturverteilung auf der Basis des aktuellen sowie der letzten N Messwertetripel $M_{k-1}$ bis $M_{k-N}$ abschätzt, dass heißt allgemein

$$Z_k=f(M_k, M_{k-1}, \ldots\ldots\ldots M_{k-N}).$$

Die Maßzahl bzw. das Maß soll nun so definiert sein, dass die Unterschreitung eines gewissen Schwellwertes S bzw. einer gewissen Schwelle eine akzeptable Verringerung des Temperaturgradienten anzeigt. Mit anderen Worten: Alle Messwertepaare $\{\eta_k, T_{30,k}\}$, für deren zugehörige Maßzahl

$$Z_k<S$$

gilt, können mit hinreichender Genauigkeit als Punkte der Viskositäts-Temperatur-Charakteristik $\eta=\eta(T)$ angesehen werden.

Physikalischer Hintergrund des vorgeschlagenen Verfahrens ist die Annahme, dass man, aufgrund der thermischen Ausgleichsvorgänge, von kleiner werdenden zeitlichen Änderungen der lokalen Temperatur auf kleinere räumliche Temperaturgradienten schließen kann. Beispielhaft wird hierfür ein besonders einfaches Beispiel für die Konstruktion von $Z_k$ angegeben. Mit der Vorschrift

$$Z_k = f(M_k, M_{k-1}) = |(T_{30,k} - T_{30,k-1}) / (t_{k-1})|$$

wird der Betrag des zeitlichen Differenzenquotienten des Temperaturmesswerts als ein Maß für die Inhomogenität der Temperatur der Flüssigkeit ermittelt. Liegt das so ermittelte Maß $Z_k$ unter einer vorgegebenen Schwelle S, so wird die Temperaturverteilung als hinreichend homogen angesehen. In diesem Beispiel wurde nur das aktuelle Messwerttripel und das Messwertetripel zeitlich davor betrachtet, das heißt, es galt N=1. Des weiteren wurden nur die Temperaturmesswerte zur Konstruktion von $Z_k$ herangezogen. Verallgemeinerungen dieses Ausdrucks, z.B. durch Bewertung der Änderung im Viskositätsmesswert - um die Temperaturänderungen am Ort des Viskositätssensors 20 indirekt zu bewerten - bzw. die Betrachtung mehrerer in der Vergangenheit liegender Wertetripel sind erfindungsgemäß ebenfalls vorgesehen.

Die vorliegende Erfindung sieht weiterhin vor, dass Messwertetripel korrigiert werden, statt diese lediglich auszufiltern. Dies ist insbesondere dann vorteilhaft durchführbar, wenn der Erwärmungs-/Abkühlungsmechanismus der Flüssigkeit sehr gut studiert und bekannt ist und die Ausbildung von entsprechenden Temperaturgradienten eingehender studiert wird. Zum Beispiel ist es erfindungsgemäß vorgesehen, aus einer gemessen zeitlichen Temperaturänderungen auf einen entsprechenden Fehler

$$\varepsilon = T_{20} - T_{30}$$

zurückzuschließen und den Messwert $T_{30}$ entsprechend zu korrigieren.

Zur Bewertung des Temperaturgradienten können auch eine Mehrzahl von Temperatursensoren 30 räumlich getrennt angeordnet werden. In diesem Fall ist anstelle des einzigen in Figur 1 dargestellten Temperatursensors 30 eine Mehrzahl solcher Temperatursensoren an verschiedenen Orten, insbesondere des Behälters 10 erfindungsgemäß vorgesehen. Somit kann die Temperatur an der Stelle des Viskositätssensors 20 beispielsweise durch Mittelung abgeschätzt werden.

**Patentansprüche**

1. Verfahren zur Messung der Viskosität einer Flüssigkeit (12) in Abhängigkeit der Temperatur der Flüssigkeit (12), wobei

    - ein Viskositätssensor (20) und
    - wenigstens ein Temperatursensor (30),

an unterschiedlichen Orten vorgesehen sind,
wobei ausgehend von zeitlich zurückliegenden Temperatur- und/oder Viskositätsmessungen auf die Viskosität der Flüssigkeit (12) am Ort des Temperatursensors (30) zu einem vorgegebenen Zeitpunkt geschlossen wird,
**dadurch gekennzeichnet, dass**
der Messwert des Viskositätssensors (20) und/oder des Temperatursensors (30)

    - verworfen wird, wenn ein Maß (Z) für die Inhomogenität der Temperatur der Flüssigkeit (12) eine vorgegebene Schwelle (S) übersteigt oder
    - in Abhängigkeit eines Maßes (Z) für die Inhomogenität der Temperatur der Flüssigkeit (12) korrigiert wird.

2. Verfahren nach Anspruch 1, **dadurch gekennzeichnet, dass** als Maß (Z) der Inhomogenität der Temperatur der Flüssigkeit (12) der zeitliche Differenzenquotient der Temperatur und/oder der Viskosität herangezogen wird.

3. Verfahren nach Anspruch 1 wobei das Maß (Z) der Inhomogenität der Temperatur der Flüssigkeit aus dem zeitlichen Verlauf der gemessenen Temperatur und/oder Viskosität der Flüssigkeit (12) ermittelt wird.

**4.** Vorrichtung zur Messung der Viskosität einer Flüssigkeit (12) in Abhängigkeit der Temperatur der Flüssigkeit (12), wobei

- ein Viskositätssensor (20) und
- wenigstens ein Temperatursensor (30),

an unterschiedlichen Orten vorgesehen sind,
wobei Mittel (40) zur Durchführung eines Verfahrens nach einem der vorhergehenden Ansprüche vorgesehen sind.

**Claims**

**1.** Method for measuring the viscosity of a liquid (12) in dependence on the temperature of the liquid (12),

- a viscosity sensor (20) and
- at least one temperature sensor (30) being provided at different locations,
the viscosity of the liquid (12) at the location of the temperature sensor (30) at a specified point in time being concluded on the basis of temperature and/or viscosity measurements taken in the past,

**characterized in that**
the measured value of the viscosity sensor (20) and/or of the temperature sensor (30)

- is discarded if a measure (Z) of the inhomogeneity of the temperature of the liquid (12) exceeds a specified threshold (S) or
- is corrected in dependence on a measure (Z) of the inhomogeneity of the temperature of the liquid (12).

**2.** Method according to Claim 1, **characterized in that** the temporal difference quotient of the temperature and/or the viscosity is used as the measure (Z) of the inhomogeneity of the temperature of the liquid (12).

**3.** Method according to Claim 1, the measure (Z) of the inhomogeneity of the temperature of the liquid being determined from the variation over time of the measured temperature and/or viscosity of the liquid (12).

**4.** Device for measuring the viscosity of a liquid (12) in dependence on the temperature of the liquid (12),

- a viscosity sensor (20) and
- at least one temperature sensor (30)

being provided at different locations,
means (40) for carrying out a method according to one of the preceding claims being provided.

**Revendications**

**1.** Procédé de mesure de la viscosité d'un liquide (12) en fonction de sa température, dans lequel

- un capteur de viscosité (20) et
- au moins un capteur de température (30)

sont prévus en différents endroits,
la viscosité du liquide (12) étant prise à l'endroit du capteur de température (30) à un moment prédéterminé à partir des mesures de température et/ou de viscosité rapportées dans le temps,
**caractérisé en ce que**
la valeur de mesure du capteur de viscosité (20) et/ou du capteur de température (30)

- est rejetée lorsqu'une mesure (Z) relative à l'inhomogénéité de la température du liquide (12) dépasse un seuil (S) prédéterminé ou
- est corrigée en fonction d'une masse (Z) relative à l'inhomogénéité de la température du liquide (12).

**2.** Procédé selon la revendication 1,
**caractérisé en ce que**
pour la mesure (Z) relative à l'inhomogénéité de la température du liquide (12), on rapproche le quotient différentiel dans le temps de la température et/ou de la viscosité.

**3.** Procédé selon la revendication 1,
selon lequel
on détermine la mesure (Z) relative à l'inhomogénéité de la température du liquide (12) à partir du tracé temporel de la température et/ou de la viscosité mesurée du liquide (12).

**4.** Dispositif de mesure de la viscosité d'un liquide (12) en fonction de la température du liquide (12), dans lequel

- un capteur de viscosité (20) et
- au moins un capteur de température (30)

sont prévus en différents endroits,
des moyens (40) pour mettre en oeuvre un procédé selon l'une quelconque des revendications précédentes étant prévus.

FIG. 1